# EUROPEAN PATENT SPECIFICATION

(11) **EP 0 707 499 B1**
(45) Date of publication and mention of the grant of the patent: **21.10.1998**
(21) Application number: 94913460.5
(22) Date of filing: 03.05.1994
(51) Int. Cl.: A61M 1/00

(54) **DOUBLE-VALVED VARIABLE PRESSURE DEVICE FOR HYDROCEPHALY**
VORRICHTUNG MIT ZWEI VENTILEN UND VERÄNDERBAREM DRUCK FÜR HYDROPHALUSBEHANDLUNG
DISPOSITIF A PRESSION VARIABLE A DEUX VALVES DESTINE AUX HYDROCEPHALIES

(43) Date of publication of application: 24.04.1996
(73) Proprietor: PHOENIX BIOMEDICAL CORP., Bridgeport, PA 19405 (US)
(72) Inventor: PAES, Newton, 04725-010-S o Paulo, SP (BR)
(74) Representative: Faber, Jean-Paul
(86) International application number: PCT/BR94/00017
(87) International publication number: WO 95/29715

(56) References cited:
- US-A- 4 621 654
- US-A- 4 675 003

## Description

This Patent of Invention relates to a **Double-valved variable pressure device for Hydrocephaly,** or more particularly to technical and functional improvements specially developped in view of featuring specific improvements to the operation of a valve for hydrocephaly used under medical conditions demanding the removal of cephalo-rachidian liquid out of the central nervous system, so proceeding in an attempt to maintain the intracranial pressure stable and proper.

An example of the prior art valves of this kind is given in US-A-4 675 003.

As already known by the specialists in this area, there are currently numberless types of apparatus for the above purpose, although they have in most cases only one valved element and, accordingly, the control of outflow and the cephalo-rachidian liquid pressure are solely and exclusively dependent on the perfect operation of only one valve, so implying some inconvenients, among which we point out: a) the proper relationship between the volume of drained liquid and the intracranial pressure is not managed; b) they produce the phenomenum of hyperdrainage (the syphon effect), with important medical complications, that are strongly described in the specialized literature.

In view of the aforementioned inconvenients, and in order to overcome them, this DOUBLE-VALVED VARIABLE PRESSURE DEVICE was conceived, wherein the cephalo-rachidian pressure and the outflow control are achieved by two valves, so translating, a new operational concept, i.e., a first valvular element determines the system's outflow until a given "minor" pressure, i.e., the intracranial pressure, propagated by means of the liquid means into the device, exerting a closing force on the first valve and, accordingly, the higher the pressure at this part of the device, the higher will be the closing of this valve, until it is fully closed, time on which the intracranial pressure is released through a second valve that, such as the former one, permits the liquid to be properly drained and, this "higher" pressure being stopped, the second valve will mandatorily closed, while the first one returns to its former "open" stage, where the cycle is once again repeated, so implying a number of technical and practical advantages, among which: a) no hyperdrainage phenomenum is allowed; b) the outflow permitted by the system is directly related to the intracranial pressure the patient is submitted to; c) the system is continuously self-adjustable by means of two valves; d) the cephalo-rachidian liquid pressure and outflow do not depend just on a valve system, but rather on two balanced systems, which renders possible to have a more precise and proper control to each patient.

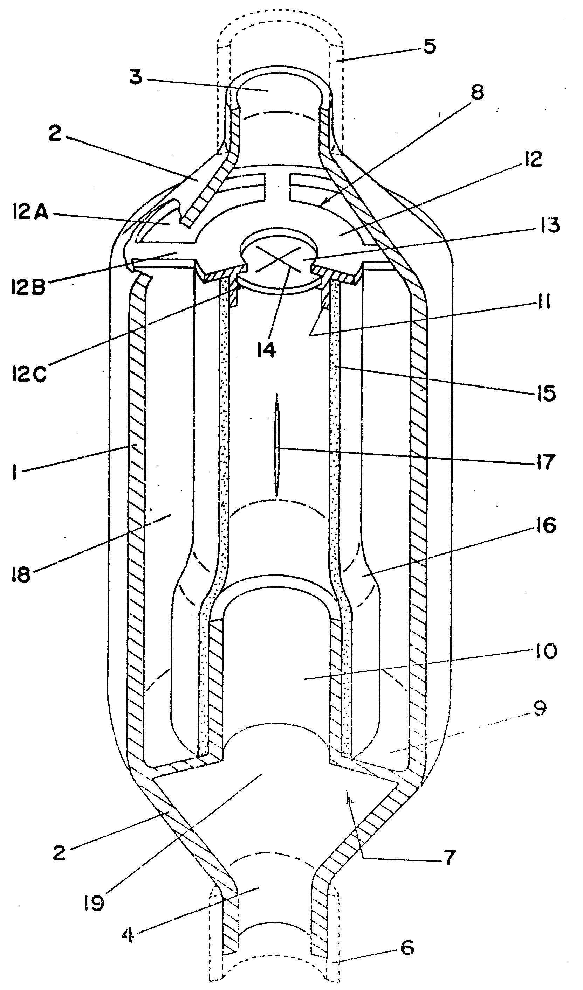

In order to provided a better understanding of the invention as described in claim 1 reference is made to the attached drawings, wherein the single figure represents a perspective view, making outstand all the internal and external constructive details of this device.

The operation of the device so described is extremely simple, i.e., the intraventricular liquid reaches the inner section of the capsule through the proximal catheter (5) and floods the first chamber (18). Under these conditions, the first outflow via for the cephalo-rachidian liquid is defined through the slot (17), wherein said tension forces permit the lengthwise slot to remain open and, consequent-ly, the cephalo-rachidian liquid outflows into chamber (19) under control, and so said slot (17), which in accordance with its area determines the effect of the system's outflow adjusting valve, since the intracranial pressure, propagated through the liquid means into the chamber (18) exerts a closing force on the tube wall (15), with the resulting variation in the slot area. This variation of area is inversely proportional to the loss of system's load, the flow being kept constant under the several pressures the system might be subject to, although, if said pressure reaches a maximum level, the slot (17) is fatally closed in full, time on which the second valve (13) starts operating and, this way, the cephalo-rachidian liquid also outflows to chamber (19), until said pressure falls again to such an extent to open the slot (17) and close the second valve again, time on which the operational cycle is started once again, with no risk of appearing the phenoloenum of hyperdrainage.

Finally, the cephalo-rachidian liquid resulting from the valvular system is drained by the distal catheter (6), which is applied into a biological cavity or an external area, according to the preference and professional experience of the physician using this prothesis.

## Claims

1. A **DOUBLE-VALVED VARIABLE PRESSURE DEVICE FOR HYDROCEPHALY,** comprising a frame in the form of a capsule made of non-deformable material (1), substantially of cylindrical shape, whose sloped ends (2) end in proximal (3) and distal (4) coupling sectors, suitable for connection to a proximal (5) and a distal (6) catheter, which constitute the input and output for the cephalo-rachidian liquid, through the capsule (1), which, in turn, is internally provided with two annular sectors, a distal (7) and a proximal (8) one, both of them made of rigid and non-deformable material, characterized in that the distal sector (7) forms a watertight closing through a flange (9), having its outer perimeter integrated to the capsule wall (1), while its inner perimeter is integrated to a tubular short coupling sector (10) coaxial with the capsule, the tubular short coupling sector, in turn, is coaxially aligned with another similar coupling sector having a smaller diameter (11), also the said similar coupling sector being integrated to a flange (12), but presenting a number of openings (12A), wherein the similar coupling sector is formed by a ring linked to the capsule wall (1) by means of several radial extensions (12B) and its inner perimeter presents a double-wall configuration (12C) and comprises a second valve, in the form of a resilient disc (13), with a centered cross-slit (14), while the first valve is defined by a tube of resilient material (15), whose inner diameter is substantially equal to the external diameter of the similar coupling sector (11), unlike the tubular short coupling sector (10), since this latter presents a diameter substantially larger than the inner diameter of the tube (15) in its unstressed position, so attributing to the tube (15) an elastic deformation (16), where said resilient tube (15) presents a longitudinal slot (17), which is kept open thanks to said deformation caused by the tubular short coupling sector (10) and the consequent tension forces appearing in the resilient tube wall at the location of the slot (17), with the resulting opening of said slot (17) so that both valves define two chambers for the cephalo-rachidian liquid, one of them defined as a retention and control chamber (18) and the other as an outflow chamber (19).

## Patentansprüche

1. Vorrichtung für veränderlichen Druck mit zwei Ventilen bei Hydrocephalie mit einem Rahmen in Form einer Kapsel aus nicht deformierbarem Werkstoff (1) mit im wesentlichen zylindrischer Gestalt, deren schräge Enden (2) in proximalen (3) und distalen (4) Koppelsektoren enden, welche zur Verbindung mit einem proximalen (5) und distalen (6) Katheter geeignet sind, die den Einlaß und den Auslaß für die cephalo-rachidische Flüssigkeit durch die Kapsel (1) bilden, welche wiederum innenseitig mit zwei ringförmigen Sektoren, einem distalen (7) und einem proximalen (8), versehen ist, die beide aus einem starren und nicht deformierbaren Werkstoff hergestellt sind, dadurch gekennzeichnet, daß der distale Sektor (7) durch einen Flansch (9) einen wasserdichten Abschluß bildet, dessen äußerer Umfang in der Kapselwand (1) integriert ist, während sein innerer Durchmesser in einen rohrförmigen, kurzen Koppelsektor (10) übergeht, der koaxial mit der Kapsel und koaxial mit einem weiteren ähnlichen Koppelsektor ist, welcher einen kleineren Durchmesser (11) hat und in einen Flansch (12) integriert ist, der aber eine Anzahl von Öffnungen (12a) aufweist, wobei der ähnliche Kopplungssektor von einem Ring gebildet ist, der mit der Kapselwand (1) mittels mehrerer radialer Verstrebungen (12b) verbunden ist, und dessen innerer Durchmesser doppelwandig (12d) konfiguriert ist und ein zweites Ventil in Form einer nachgiebigen Scheibe (13) mit einem mittigen Kreuzschlitz (14) enthält, während das erste Ventil von einem Rohr aus nachgiebigem Material (15) gebildet ist, dessen innerer Durchmesser im wesentlichen gleich dem Außendurchmesser des ähnlichen Kopplungssektors (11) aber nicht entsprechend dem rohrförmigen kurzen Kopplungssektor (10) ist, weil dieser einen im wesentlichen größeren Durchmesser aufweist als der Innendurchmesser des Rohres (15) in dessen nicht gedehntem Zustand, so daß er dem Rohr (15) eine elastische Deformation (16) aufprägt, indem das nachgiebige Rohr (15) einen länglichen Schlitz (17) aufweist, welcher aufgrund dieser Deformation offen gehalten wird, die von dem rohrförmigen kurzen Kopplungssektor (10) und den von ihm erzeugten Dehnungskräften bewirkt wird, welche in der Wand des nachgiebigen Rohrs am Ort des Schlitzes (17) mit dem Resultat der Öffnung des Schlitzes (17) auftreten, so daß beide Ventile zwei Kammern für die cephalo-rachidische Flüssigkeit begrenzen, von denen eine als Rückhalte- und Steuerungskammer (18) und die andere als Ausflußkammer (19) definiert ist.

## Revendications

1. Dispositif à pression variable à deux valves destiné aux hydrocéphalies, comprenant un châssis sous forme de capsule fabriquée en matériau (1) non déformable, sensiblement de forme cylindrique, dont les extrémités inclinées (2) se terminent en secteurs de couplage proximal (3) et distal (4), appropriés pour être connectés à un cathéter proximal (5) et à un cathéter distal (6), qui constituent l'orifice d'admission et l'orifice de sortie du liquide céphalo-rachidien, à travers la capsule (1), qui, à son tour, est munie à l'intérieur de deux secteurs annulaires, dont un secteur distal (7) et un secteur proximal (8), les deux étant fabriqués en matériau rigide et non déformable, caractérisé en ce que le secteur distal (7) forme une fermeture étanche au moyen d'une bride (9), en ayant son périmètre extérieur intégré à la paroi de la capsule (1), tandis que son périmètre intérieur est intégré à un secteur de couplage (10) court et tubulaire coaxial à la capsule, le secteur de couplage court et tubulaire, à son tour, étant coaxialement aligné avec l'autre secteur de couplage similaire, ayant un diamètre inférieur (11), ledit secteur de couplage similaire étant également intégré à une bride (12), mais présentant un certain nombre d'ouvertures (12A), dans lequel le secteur de couplage similaire est formé par un anneau raccordé à la paroi de la capsule (1), au moyen de plusieurs extensions radiales (12B) et son périmètre intérieur présente une configuration en double paroi (12C) et comprend une seconde valve, sous la forme d'un disque élastique (13), avec une fente croisée centrée (14), tandis que la première valve est définie par un tube en matériau élastique (15), dont le diamètre intérieur est sensiblement égal au diamètre extérieur du secteur de couplage similaire (11), contrairement au secteur de couplage (10) court et tubulaire, puisque ce dernier présente un diamètre sensiblement plus important que le diamètre intérieur du tube (15) dans sa position de repos, conférant au tube (15) une déformation élastique (16) où ledit tube élastique (15) présente une fente longitudinale (17), qui est maintenue ouverte grâce à ladite déformation provoquée par le secteur de couplage (10) court et tubulaire et les forces de tension qui en découlent apparaissant dans la paroi du tube élastique à l'emplacement de la fente (7), avec l'ouverture de ladite fente (17) qui en résulte de telle sorte que les deux valves définissent deux chambres pour le liquide céphalo-radichien, dont une définie comme chambre de rétention et de contrôle (18) et l'autre, comme chambre d'évacuation (19).
